# FASCICULE DE BREVET EUROPEEN

(11) **EP 0 141 693 B1**
(45) Date de publication et mention de la délivrance du brevet: **16.02.1994**
(21) Numéro de dépôt: 84401734.3
(22) Date de dépôt: 29.08.1984
(51) Int. Cl.: A61F 13/02

(54) **Pansement compressif hémostatique**
Blutstillender Druckverband
Hemostatic compressive dressing

(30) Priorité: 29.08.1983 FR 8313948
(43) Date de publication de la demande: 15.05.1985
(73) Titulaire: HOSPAL AG, 4008 Basel (CH); HOSPAL INDUSTRIE, F-69330 Meyzieu (FR)
(72) Inventeur: Buisson, Philippe, F-38090 Villefontaine (FR)

(56) Documents cités:
- EP-A- 0 067 622
- GB-A- 1 588 933
- US-A- 3 368 911
- US-A- 4 162 672

## Description

La présente invention concerne un pansement compressif hémostatique selon le préambule le dela revendication 1 et connu du document EP-A-67622. La plupart des pansements actuellement connus exerçant une action mécanique (compressive) au moyen d'un tampon pour provoquer l'arrêt d'un saignement au niveau d'hémorragies en particulier, par exemple, après le retrait d'une aiguille ayant servi à une intervention dans une veine ou dans une artère présentent un certain nombre d'inconvénients.

C'est ainsi qu'ils provoquent l'écrasement plus ou moins important du conduit sanguin et/ou en obturent la lumière, source de l'hémorragie, empêchant ainsi la libre circulation du sang dans ledit conduit. Un autre inconvénient réside dans le fait qu'au moment de leur retrait, dans bon nombre de cas, ils n'évitent pas la réactivation de l'hémorragie par suite de l'adhérence de thrombus sur le pansement, remettant ainsi à vif la lumière du vaisseau sanguin ou les lèvres d'une plaie. De tels pansements nécessitent aussi des temps d'hémostase relativement longs, surtout si l'action compressive recherchée se traduit en même temps par un certain écartement de la lumière du conduit sanguin ou des lèvres d'une plaie.

Or, la présente invention fournit un pansement obviant aux inconvénients ci-dessus et caractérisé en ce que le corps est rigide et en ce qu'il supporte sur une face opposée à la lande de fination une couche de collagène.

Suivant une caractéristique avantageuse, ladite bande est munie de moyens permettant le contrôle et le maintien de l'intensité de serrage ou de comcompression dudit corps sur le point ou la zone d'hémorragie.

Suivant d'autres caractéristiques :
- ledit corps à action compressive est en un matériau non absorbant du type métal mou, matière plastique ou verre synthétique incassable ou analogue ;
- ledit corps affecte avantageusement la forme d'une calotte sphérique ;
- la couche de collagène est avantageusement une pastille de collagène, de préférence de collagène lyophilisé, fixé par collage sur ledit corps à action compressive.

D'autres caractéristiques et avantages de l'invention ressortiront plus clairement de la description qui va suivre faite en regard des dessins annexés sur lesquels :
- la figure 1 est une vue en élévation-coupe d'un corps d'un pansement selon l'invention dans sa forme la plus simple ;
- la figure 2 est une vue en élévation-coupe du pansement selon la figure 1, monté sur un support et,
- la figure 3 est une vue en plan correspondant à la figure 2.

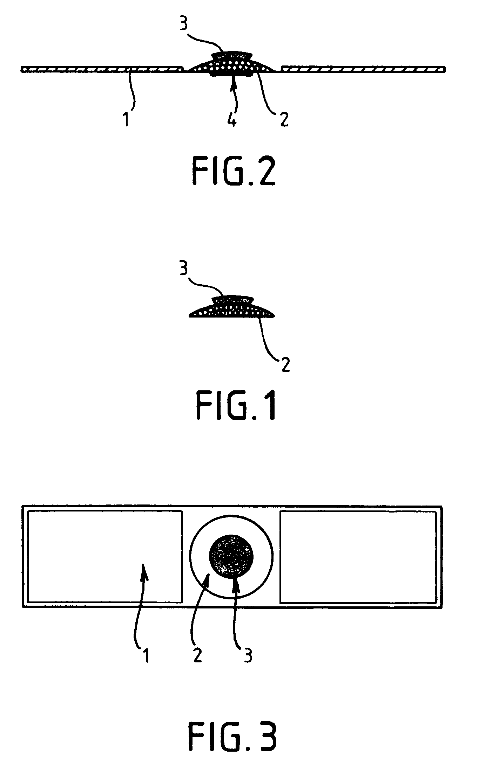

En se référant à la figure 1, uncorps (2) d'un pansement selon l'invention est un corps rigide 2 en matériau non absorbant choisi par exemple parmi les matériaux en métal mou, les matières plastiques, les verres, les verres synthétiques et analogues. Il aura toute forme appropriée, de préférence la forme d'une calotte sphérique, sur laquelle est appliquée une couche 3 de collagène. Ce matériau pourra être sous forme de poudre, de gel ou d'une pastille. Le collagène est, de préférence, le collagène lyophilisé sous forme d'une pastille, avantageusement collée sur le corps 2. L'action compressive combinée à l'action due aux propriétés hémostatiques du collagène permet de respecter le conduit ou le vaisseau sanguin, par exemple après le retrait d'une aiguille ayant servi à une intervention dans une veine, dans une artère ou dans un pontage, sans risque d'écrasement, en laissant ainsi librement circuler le sang. On constate alors qu'en mettant ainsi à profit cette double action, on obtient un temps d'hémostase bien moins long qu'avec les pansements compressifs connus présentant les inconvénients rappelés précédemment.

En se référant aux figures 2 et 3, un pansement selon l'invention a aussi une bande 1, de forme et de dimensions apppropriées, faite en tout matériau connu, habituellement utilisé dans le domaine des pansements, bande portant dans sa partie médiane, soit par fixation, soit par collage, soit formé au sein du matériau constitutif de la bande elle-même, le corps 2 disposé en relief et portant lui-même le matériau hémostatique 3.

On peut également prévoir sur la face externe de la bande 1, une pastille repérée 4, par exemple colorée en tout matériau approprié permettent de siteer exactement l'axe dela couche de collagène 3 pour en faciliter la pose sur le point ou la zone à laquelle elle est destinée.

Le matériau constitutif de la bande 1 peut être par exemple une matière textile, tissée ou non-tissée, une matière plastique simple et analogue. Dans ce dernier cas, on conçoit fort bien que le corps en relief 2 pourra être formé en même temps que la bande par tout procédé de moulage, de thermoformage, de coulage, voire d'extrusion, connu dans le domaine des matières plastiques. Si elle n'est pas rendue adhésive, pour en faciliter la pose ou le maintien en position sur le membre ou l'organe en cause, ou si ce caractère adhésif est considéré, dans certains cas, comme pouvant être nuisible, la bande 1 comportera des moyens connus en soi; permettant après avoir entouré l'organe ou le membre, de régler le serrage et, de ce fait, l'intensité de l'action compressive, moyens tels que crantages, surfaces adhérentes se recouvrant, etc. Ces moyens ne sont pas illustrés sur les dessins.

On comprendra aisément la façon dont on pourra se servir d'un tel pansement. Il suffira de le sortir de son emballage (ne faisant pas partie de la présente invention) et d'appliquer le matériau hémostatique sur la zone ou le point où l'arrêt est recherché, en faisant adhérer la bande sur la peau, si cette bande est adhésive, ou bien, dans la mesure du possible, en entourant le membre en cause de la bande et en se servant des moyens de serrage et/ou de maintien en position dont elle est munie pour parfaire l'action compressive.

Les actions simultanées du corps en relief 2 (action compressive) et celles de la couche de collagène 3 auront pour effet, comme précisé précédemment, d'arrêter le saignement de façon plus rapide que tout autre pansement comportant généralement un corps absorbent exerçant ou non une action compressive, corps absorbant dont le retrait présente souvent l'inconvénient de provoquer un nonveau saignement.

Il va de soi que la présente invention n'a été décrite qu'à titre purement illustratif et nullement limitatif et que toute modification pourra y être apportée par l'homme de l'art sans sortir de son cadre comme il est définie dans les revendications.

## Revendications

1. Pansement destiné à être appliqué sur la blessure laissée par le retrait d'une aiguille après son introduction dans un vaisseau sanguin, comprenant une bande de fixation (1) souple ayant une face adhésive, et un corps (2) formant relief du côté de la face adhésive, caractérisé en ce que le corps (2) est rigide et en ce qu'il supporte sur une face opposée à la bande de fixation (1) une couche de collagène (3).

2. Pansement selon la revendication 1, caractérisé en ce que le corps (2) est non absorbant.

3. Pansement selon une des revendications 1 et 2, caractérisé en ce que la face du corps (2) supportant la couche de collagène (3) a la forme d'une calotte sphérique.

4. Pansement selon une des revendications 1 à 3, caractérisé en ce que le corps (2) est en matière plastique.

5. Pansement selon une des revendications 1 à 4, caractérisé en ce que la bande de fixation (1) est en matière plastique.

## Claims

1. Dressing intended to be applied to the wound left by the withdrawal of a needle after its introduction into a blood vessel, comprising a flexible fixing strip (1) having an adhesive surface, and a body (2) forming a relief on the side of the adhesive surface, characterized in that the body (2) is rigid, and in that it supports, on a surface opposite the fixing strip (1), a layer of collagen (3).

2. Dressing according to Claim 1, characterized in that the body (2) is non-absorbent.

3. Dressing according to one of Claims 1 and 2, characterized in that the surface of the body (2) supporting the layer of collagen (3) has the form of a segment of a sphere.

4. Dressing according to one of Claims 1 to 3, characterized in that the body (2) is made of plastic material.

5. Dressing according to one of Claims 1 to 4, characterized in that the fixing strip (1) is made of plastic material.

## Patentansprüche

1. Verband, der bestimmt ist, auf die Wunde aufgelegt zu werden, die durch das Herausziehen einer Nadel nach ihrer Einführung in ein Blutgefäß verbleibt, umfassend ein flexibles Fixationsband (1), das eine haftende Seite aufweist, und einen Körper (2), der eine Erhöhung neben der haftenden Seite bildet, dadurch gekennzeichnet, daß der Körper (2) steif ist und daß er auf einer zu dem Fixationsband (1) abgewandten Seite eine Collagenschicht (3) trägt.

2. Verband nach Anspruch 1, dadurch gekennzeichnet, daß der Körper (2) nicht-absorbierend ist.

3. Verband nach einem der Ansprüche 1 und 2, dadurch gekennzeichnet, daß die die Collagenschicht (3) tragende Seite des Körpers (2) die Form einer sphärischen Kalotte aufweist.

4. Verband nach einem der Ansprüche 1 bis 3, dadurch gekennzeichnet, daß der Körper (2) aus Kunststoffmaterial besteht.

5. Verband nach einem der Ansprüche 1 bis 4, dadurch gekennzeichnet, daß das Fixationsband (1) aus Kunststoffmaterial besteht.
